# EUROPÄISCHE PATENTSCHRIFT

(11) **EP 1 092 804 B1**
(45) Veröffentlichungstag und Bekanntmachung des Hinweises auf die Patenterteilung: **10.08.2005**
(21) Anmeldenummer: 99120573.3
(22) Anmeldetag: 16.10.1999
(51) Int. Cl.: D06M 13/256, D06M 13/148, D06M 13/17, D06M 15/53, D06M 13/262, C11D 1/825, C11D 3/34, C11D 3/20

(54) **Zusammensetzung für die Vorbehandlung von Fasermaterialien**
Composition for the pretreatment of textiles
Composition pour le prétraitement de textiles

(43) Veröffentlichungstag der Anmeldung: 18.04.2001
(73) Patentinhaber: Ciba Spezialitätenchemie Pfersee GmbH, 86462 Langweid a.L. (DE)
(72) Erfinder: Prozzo, Biancamaria Dr., 86153 Augsburg (DE); Seifert, Peter, 86485 Biberach (DE)

(56) Entgegenhaltungen:
- EP-A- 0 354 344
- EP-A- 0 491 531
- EP-A- 0 696 661
- US-A- 5 858 955

## Beschreibung

Die Erfindung betrifft eine Zusammensetzung, welche mindestens Wasser, ein organisches Sulfonat oder einen mehrwertigen Alkohol und daneben einen ethoxilierten Alkohol und einen ethoxilierten/propoxilierten Alkohol enthält. Sie betrifft ferner die Verwendung solcher Zusammensetzungen für die Behandlung von Fasermaterialien.

Fasermaterialien in Form textiler Flächengebilde, wie z.B. Gewebe, müssen im Normalfall einer sog. Vorbehandlung unterzogen werden, bevor sie gefärbt werden. Die Vorbehandlung dient u.a. dazu, eine störungsfreie gleichmäßige Färbung zu erzielen. Je nach Vorgeschichte und Provenienz der textilen Flächengebilde oder maschinellen Gegebenheiten kann die Vorbehandlung unter anderem die Maßnahmen Entschlichten, Entfetten/Reinigen und Bleichen der Textilien umfassen, wobei diese Maßnahmen getrennt voneinander durchgeführt werden können. Es ist jedoch im Einzelfall auch möglich, mehrere dieser Maßnahmen in einem einzigen Prozeß ablaufen zu lassen, um Kosten zu sparen.
Um die Zwecke der Vorbehandlung zu erfüllen, ist es wichtig, daß das Textil sowohl bei Beginn der Vorbehandlung als auch nach der Vorbehandlung eine gute Benetzbarkeit durch wäßrige Systeme aufweist, also eine gute Primärbenetzbarkeit und eine gute Wiederbenetzbarkeit. Letztere dient dazu, einen problemlosen Färbeprozeß zu ermöglichen.
Um die Forderungen zu erfüllen, welche an einen geeigneten Vorbehandlungsprozeß zu stellen sind, verwendet man für die Vorbehandlung verschiedene chemische Produkte. Zu diesen können je nach Aufgabenstellung Netzmittel, Waschmittel, Enzyme, Bleichmittel, Stabilisatoren, Komplexiermittel usw. gehören. Besondere Bedeutung kommt hierbei Produkten zu, welche eine gute Wiederbenetzbarkeit der textilen Flächengebilde nach der Vorbehandlung bewirken müssen. Textilien mit guter Wiederbenetzbarkeit fördern die Gleichmäßigkeit der Anfärbung beim nachfolgenden Färbeprozeß. Dabei ist es wichtig, daß die Produkte, die eine gute Wiederbenetzbarkeit herbeiführen, keine oder nur eine unwesentliche Erhöhung der Schaumbildungstendenz in Bädern bewirken, in welchen die Vorbehandlungsprodukte enthalten sind. Diese Forderung spielt vor allem auch eine große Rolle, wenn die Vorbehandlung als diskontinuierlicher Prozeß, z.B. in sogenannten Jet-Anlagen durchgeführt wird, in denen sich erhöhte Schaumbildung sehr störend auswirken kann und in denen die Gefahr erhöhter Schaumbildung stärker ist als bei anderen Verfahren. Man verlangt häufig von den für die Vorbehandlung ver wendeten Produkten eine geringe Schaumbildungstendenz, weil es vielfach unerwünscht ist, eine erhöhte Schaumbildungstendenz durch Zusatz von Antischaummitteln wie Silikonen zu unterdrücken.

Nachdem klassische Vorbehandlungsschritte wie Entschlichten, Entfetten/Reinigen, Bleichen in einer Reihe von Fällen zu einem kombinierten Vorbehandlungsprozeß zusammengefaßt werden, stellte sich auch die Forderung nach Zusammensetzungen, welche für solche kombinierten Vorbehandlungsprozesse verwendet werden können. Die Zusammensetzungen müssen wäßrige Systeme sein, welche eine gute Benetzbarkeit der textilen Ware zu Beginn der Vorbehandlung bewirken, aber auch eine gute Hydrophilie nach deren Ende. Diese gute Hydrophilie führt zu einer guten Wiederbenetzbarkeit, die im Hinblick auf den Färbeprozeß erforderlich ist.

Produkte, welche sich für Vorbehandlung von Fasermaterialien in Form textiler Flächengebilde eignen, sind bekannt.
Die EP-A 98 803 beschreibt Pfropfpolymerisate, welche einen hydrophoben Anteil, einen daran gebundenen Polyglykoletheranteil und einen hydrophilen aufgepfropften Bestandteil enthalten. Der gepfropfte hydrophile Bestandteil kann hierbei ionische Gruppen wie Sulfonat- oder Carboxylatreste bzw. die entsprechenden Säuregruppen enthalten.
Aus der EP-A 462 059 gehen Textilhilfsmittel hervor, welche einen alkoxilierten, ggf. endständig veretherten Alkohol, ein Umsetzungsprodukt aus einem solchen Alkohol und einer sauren Verbindung und gegebenenfalls ein Hydrotropiermittel enthalten. Das Umsetzungsprodukt aus alkoxiliertem Alkohol und einer sauren Verbindung kann ein Sulfonat sein.
Gemäß WO 92/15664 enthalten Textilbehandlungsmittel Homopolymere von ungesättigten Sulfon- oder Carbonsäuren, ein nichtionisches Tensid und ggf. ein Hydrotropiermittel. Das Tensid ist wie im Fall der oben zitierten EP-462 059 ein Alkoxilat eines aliphatischen Alkohols, wobei das Alkoxilat am anderen Ende an Stelle von Wasserstoff auch einen organischen Rest aufweisen kann. Solche Tenside und ihre Verwendung in der Textilbehandlung gehen auch aus der EP-A 420 802 hervor.
Die EP-A 360 736 beschreibt Zusammensetzungen für die Vorbehandlung von Textilien, welche eine Phosphorverbindung, ein Mischpolymerisat mit einem hydrophilen und einem hydrophoben Bestandteil, ein nichtionogenes Tensid und ein Alkalimetallhydroxid enthalten.

Die in den oben genannten Dokumenten beschriebenen Zusammensetzungen weisen, obwohl sie sich prinzipiell für die Textilvorbehandlung eignen, noch nicht in jeder Beziehung optimale Eigenschaften auf. Insbesondere bereitet bei Zusammensetzungen für die Vorbehandlung von Textilien die Wiederbenetzbarkeit der Ware nach beendeter Vorbehandlung in einer Reihe von Fällen Probleme. Eine gute Wiederbenetzbarkeit, basierend auf einem ausreichenden Grad von Hydrophilie, muß nach der Vorbehandlung erreicht werden, um die Färbeeigenschaften des Textils nicht negativ zu beeinflussen. Nun ist es im Prinzip zwar möglich, die Wiederbenetzbarkeit durch Art und Menge der verwendeten Tenside in der Vorbehandlung zu verbessern. Es hat sich jedoch gezeigt, daß bei einer entsprechenden Optimierung der Wiederbenetzbarkeit häufig die Primärbenetzbarkeit verschlechtert und/oder die Schaumbildungstendenz deutlich erhöht wird. Die angesprochene Primärbenetzbarkeit bedeutet die Benetzbarkeit des textilen Flächengebildes beim Beginn der Vorbehandlung, also beim ersten Kontakt der Ware mit der Vorbehandlungsflotte. Diese Primärbenetzbarkeit muß ein bestimmtes Mindestniveau erreichen, damit die Vorbehandlung störungsfrei ablaufen kann. Verwendet man nun Zusammensetzungen, welche zu einer guten Wiederbenetzbarkeit führen, also einer guten Benetzbarkeit nach der Vorbehandlung, so tritt in vielen Fällen eine ungenügende Primärbenetzbarkeit auf. Versuche, durch entsprechend hohe Mengen an Tensiden sowohl Primärbenetzbarkeit als auch Wiederbenetzbarkeit zu optimieren, führen dagegen häufig zu erhöhter Schaumbildungstendenz. Diese macht sich vor allem auch bei Diskontinue-Verfahren zur Vorbehandlung störend bemerkbar, z.B. bei diskontinuierlicher Vorbehandlung in Jet-Apparaturen. Eine Dämpfung des Schaums mittels Silikonen ist zwar möglich, aber in vielen Fällen unerwünscht.

Die Aufgabe der vorliegenden Erfindung bestand darin, eine Zusammensetzung zu entwickeln, welche sich ausgezeichnet für die Vorbehandlung von Fasermaterialien, insbesondere textilen Flächengebilden, eignet, zu guter Primärbenetzbarkeit bei guter Wiederbenetzbarkeit der textilen Ware führt und die auch ohne Verwendung hocheffektiver Antischaummittel wie Silikone keine unakzeptabel hohe Schaumbildungstendenz bewirkt.

Die Aufgabe wurde gelöst durch eine Zusammensetzung, welche mindestens die Komponenten A, B, C und D enthält
wobei Komponente A entweder ein Sulfonat der Formel (I) 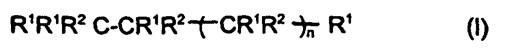 ist,
worin n eine Zahl von 0 bis 8 bedeutet, alle Reste R¹ unabhängig voneinander für Wasserstoff, einen Alkylrest mit 1 bis 4 Kohlenstoffatomen oder einen unsubstituierten oder durch einen Rest der Formel SO₃^{Θ}M^{⊕} substituierten Phenylrest stehen und alle Reste R² unabhängig voneinander für R¹ oder für einen Rest der Formel -SO₃^{Θ}M^{⊕} stehen, wobei Komponente A jedoch mindestens einen Rest der Formel SO₃^{Θ}M^{⊕} enthält und M für Na, K, oder NH₄ steht,
oder wobei Komponente A ein mehrwertiger aliphatischer Alkohol mit 2 bis 12 Kohlenstoffatomen, vorzugsweise mit 4 bis 10 Kohlenstoffatomen, ist,
wobei Komponente B ein ethoxilierter Alkohol der Formel (II) oder ein Gemisch solcher Alkohole 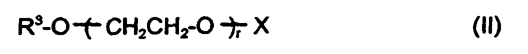 ist,
worin r eine Zahl von 1 bis 8, vorzugsweise von 2 bis 7 ist,
wobei Komponente C ein Alkoxilat der Formel (III) oder ein Gemisch solcher Alkoxilate 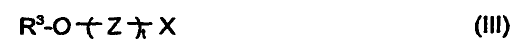 ist,
worin t eine Zahl von 4 bis 30, vorzugsweise von 6 bis 18, ist, 20 bis 80 % aller anwesenden Gruppen Z für -CH₂CH₂-O- und 80 bis 20 % aller anwesenden Gruppen Z für -CHR⁴-CHR⁵-O-stehen, wobei jeweils einer der Reste R⁴ und R⁵ für Wasserstoff und der andere für CH₃ steht, wobei sowohl in Komponente B als auch in Komponente C R³ für einen linearen oder verzweigten Alkylrest mit 4 bis 20, vorzugsweise 8 bis 18, Kohlenstoffatomen steht und 50 bis 100 %, vorzugsweise 100 %, aller anwesenden X für Wasserstoff und 0 bis 50 %, vorzugsweise 0 %, aller anwesenden Reste X für einen Methyl-, Ethyl- oder Phenylrest stehen,
und wobei Komponente D Wasser ist.

Diese oben genannte spezielle Kombination von Komponenten löst überraschenderweise eine Reihe von Problemen, welche Formulierungen aus dem Stand der Technik bei der Textilvorbehandlung verursachen. Erfindungsgemäße Zusammensetzungen der genannten Art eignen sich sehr gut für die Vorbehandlung von textilen Flächengebilden auch in den Fällen, wo mehrere der eingangs genannten Vorbehandlungsschritte in ein kombiniertes Verfahren zusammengefaßt werden. Wenn sie auch für Bleichprozesse eingesetzt werden sollen, bei denen Wasserstoffperoxid verwendet wird, empfiehlt es sich allerdings, wenn die Zusammensetzungen noch zusätzlich eine Komponente E der unten näher erläuterten Art enthalten. Die erfindungsgemäßen Zusammensetzungen besitzen Vorteile insbesondere auch im Fall ihrer Verwendung für diskontinuierliche Vorbehandlungsverfahren, z.B. in sog. Jet-Anlagen. Hierfür eignen sie sich in besonderer Weise wegen ihrer geringen Schaumbildungstendenz. Sie vermitteln den Textilien eine hohe Hydrophilie nach der Vorbehandlung, was sich in einer ausgezeichneten Wiederbenetzbarkeit ausdrückt. Die Primärbenetzbarkeit der Textilien, also die erste Benetzbarkeit durch die Vorbehandlungsflotte bei Beginn der Vorbehandlung, ist ebenfalls sehr gut.

Erreicht werden die genannten Vorteile dann, wenn die in Anspruch 1 und oben festgelegten Angaben über die Komponenten der erfindungsgemäßen Zusammensetzungen eingehalten werden.

Komponente A ist entweder ein Sulfonat der Formel (I)

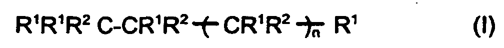

Hierbei bedeutet n eine Zahl von 0 bis 8, eine bevorzugte Ausführungsform besteht darin, daß n eine Zahl von 1 bis 3 ist. Alle Reste R¹ stehen unabhängig voneinander für Wasserstoff, einen linearen oder verzweigten Alkylrest mit 1 bis 4 Kohlenstoffatomen oder einen Phenylrest, der entweder unsubstituiert ist oder durch einen Sulfonatrest der Formel -SO₃^{Θ}M^{⊕} substituiert ist. Alle Reste R² stehen unabhängig voneinander für einen Rest R¹ der oben genannten Art oder für einen Sulfonatrest der Formel -SO₃^{Θ}M^{⊕}. Wenn Komponente A eine Verbindung der Formel (I) ist, muß sie jedoch mindestens einen Sulfonatrest -SO₃^{Θ}M^{⊕} enthalten, d.h. entweder muß mindestens einer aller anwesenden Reste R² für -SO₃^{Θ}M^{⊕} stehen oder mindestens einer aller anwesenden Reste R¹ muß ein Phenylrest sein, der einen Rest -SO₃^{Θ}M^{⊕} als Substituenten trägt. Enthält Komponente A einen oder mehrere durch -SO₃^{Θ}M^{⊕} substituierte Phenylreste, so können sich die -SO₃^{Θ}M^{⊕}-Gruppen jeweils in beliebiger Stellung am aromatischen Ring befinden. In den Resten -SO₃^{⊖}M^{⊕} steht M^{⊕} jeweils für ein Natrium-, Kalium- oder Ammoniumion. Somit kann Komponente A ein rein aliphatischer oder ein aliphatisch-aromatischer Kohlenwasserstoff sein, in dem eine oder mehrere -C-H-Bindungen durch -SO₃^{Θ}M^{⊕}-Bindungen ersetzt sind.
In einer bevorzugten Ausführungsform erfindungsgemäßer Zusammensetzungen ist Komponente A ein Sulfonat der oben genannten Formel (I), worin mindestens einer aller anwesenden Reste R² für -SO₃^{Θ}M^{⊕} steht. In diesem Fall kann es von Vorteil sein, wenn keiner der anwesenden Reste R¹ eine Sulfonatgruppe enthält.
Besonders gut geeignet für die Vorbehandlung von Textilien sind erfindungsgemäße Zusammensetzungen, in denen Komponente A ein Sulfonat der Formel (IV) ist 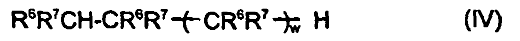 Hierin steht w für eine Zahl von 1 bis 3, einer der Reste R⁶ steht für einen unsubstituierten Phenylrest, alle übrigen Reste R⁶ stehen für Wasserstoff, einer der Reste R⁷ steht für SO₃^{Θ}M^{⊕} und alle übrigen Reste R⁷ stehen für Wasserstoff. Der einzige anwesende Phenylrest und der einzige anwesende Sulfonatrest können hierbei für einen beliebigen der Reste R⁶ bzw. R⁷ der Formel (IV) stehen. Als besonders gut geeignet als Komponente A erfindungsgemäßer Zusammensetzungen haben sich Natriumcumolsulfonat und Kaliumcumolsulfonat erwiesen.

An Stelle eines Sulfonats der Formel (I) kann die Komponente A erfindungsgemäßer Zusammensetzungen auch ein mehrwertiger aliphatischer Alkohol mit 2 bis 12, vorzugsweise mit 4 bis 10 Kohlenstoffatomen und einer linearen oder verzweigten Kohlenstoffkette, sein. Natürlich können erfindungsgemäße Zusammensetzungen auch Gemische von Sulfonaten der Formel (I) und von solchen mehrwertigen Alkoholen enthalten. Falls Komponente A kein Sulfonat ist, sondem ein mehrwertiger Alkohol, so besteht eine bevorzugte Ausführungsform erfindungsgemäßer Zusammensetzungen darin, daß Komponente A ein zweiwertiger oder dreiwertiger aliphatischer Alkohol mit linearer oder verzweigter Alkylkette mit 4 bis 8 Kohlenstoffatomen ist. Besonders geeignet als Komponente A sind 1.5-Pentandiole, in welchen an einem der C-Atome 2 bis 4 eine Methylgruppe als Substituent vorhanden ist.

Komponente A muß in erfindungsgemäßen Zusammensetzungen enthalten sein. Es hat sich nämlich gezeigt, daß Zusammensetzungen, welche zwar Wasser, eine Komponente B und eine Komponente C enthalten, aber keine Komponente A, noch nicht optimal für die Vorbehandlung von Textilien geeignet sind. Das Problem kann hier einerseits die Wiederbenetzbarkeit der textilen Ware nach Durchführung der Vorbehandlung sein: Mit üblichen Mengen an Komponenten B und C in den Flotten genügt die Wiederbenetzbarkeit vielfach nicht den hohen Anforderungen, wenn keine Komponente A anwesend ist. Das Problem Wiederbenetzbarkeit wäre im Prinzip lösbar durch Erhöhung der Menge an Komponente B und/oder C. Es hat sich aber gezeigt, daß hierfür so hohe Mengen erforderlich wären, daß die Kosten sehr hoch werden und vor allem, daß dann das Schaumproblem nicht mehr ohne Einsatz hocheffektiver Antischaummittel wie Silikone zu beherrschen ist. Andererseits können sich Silikone ungünstig auf das Färbeverhalten auswirken und sind deshalb in vielen Fällen unerwünscht. Ein anderes Problem, das bei Abwesenheit einer Komponente A auftreten kann, besteht darin, daß vielfach keine stabile flüssige, wäßrige Formulierung erhalten wird, die sich für die Behandlung von Fasermaterialien verwenden läßt und/oder daß die Formulierungen zu stark zum Schäumen neigen. Bei Zusammensetzungen, welche eine Komponente B und eine Komponente C und eine Komponente D enthalten, aber keine Komponente A, kann der Fall auftreten, daß sie in Form eines Gels vorliegen, das sich nicht für Vorbehandlungsverfahren eignet. Dieses Problem kann bei Abwesenheit einer Komponente A zusätzlich auch dann auftreten, wenn die oben genannte mangelnde Wiederbenetzbarkeit im Einzelfall kein Problem wäre. Durch die Verwendung einer Komponente A zusammen mit einer Komponente B und einer Komponente C und Wasser jedoch werden die hier angesprochenen Schwierigkeiten beseitigt.

Als Komponente A geeignete Sulfonate und mehrwertige Alkohole sind im Handel erhältlich, z.B. Na-cumolsulfonat, oder lassen sich nach bekannten chemischen Methoden herstellen. An Stelle einer einzigen Verbindung der Formel (I) bzw. (IV) kann Komponente A auch ein Gemisch solcher Verbindungen sein oder ein Gemisch mehrwertiger Alkohole.

Komponente B erfindungsgemäßer Zusammensetzungen ist in erster Linie verantwortlich für die Primärbenetzbarkeit und die Wiederbenetzbarkeit der textilen Flächengebilde zu Beginn und nach Ende der Vorbehandlung. Die Vorbehandlungsflotte soll das Textil schnell und gleichmä-ßig benetzen, damit der Zweck der Vorbehandlung optimal erreicht werden kann. Es hat sich gezeigt, daß Zusammensetzungen, welche eine Komponente A und eine Komponente C, aber keine Komponente B der genannten Art enthalten, zu einer im Normalfall schlechteren Primärbenetzbarkeit führen. Auch die Wiederbenetzbarkeit der Fasermaterialien nach Abschluß der Vorbehandlung kann auf einem zu niedrigen Niveau liegen, falls keine Komponente B in den Zusammensetzungen enthalten ist.

Komponente B ist ein ethoxilierter Alkohol der Formel (II) oder ein Gemisch solcher Alkohole

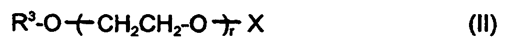

Hierin bedeutet r eine Zahl von 1 bis 8, vorzugsweise von 2 bis 7. Normalerweise ist Komponente B ein Gemisch ethoxilierter Alkohole, da bei Ethoxilierungsreaktionen wie Umsetzung von Alkoholen mit Ethylenoxid Produktgemische entstehen. Solche Gemische sind dadurch charakterisiert, daß der Wert von r (Ethoxilierungsgrad) eine gewisse Verteilung aufweist. Damit ein Gemisch ethoxilierter Produkte der Formel (II) als Komponente B erfindungsgemäßer Zusammensetzungen geeignet ist, darf der im Gemisch vorliegende häufigste Wert von r keinesfalls größer sein als 8. Es ist von Vorteil, wenn bei nicht mehr als 20 % der Moleküle des Gemischs der Wert von r größer als 8 ist, vorzugsweise ist bei mindestens 80 % der Moleküle r nicht größer als 7. Es hat sich gezeigt, daß ethoxilierte Alkohole der Formel (II), bei denen r größer als 8 ist bzw. bei denen mehr als 20 % der Moleküle des Gemischs einen Wert von r aufweisen, der größer als 8 ist, nicht als Komponente B erfindungsgemäßer Zusammensetzungen geeignet sind. Der Grund liegt darin, daß mit steigenden Werten von r die Schaumbildungstendenz zunimmt und bei größeren Werten als 8 ein unakzeptables Niveau erreichen kann. In Formel (II) steht R³ für einen linearen oder verzweigten Alkylrest mit 4 bis 20 Kohlenstoffatomen. Besonders geeignet als Komponente B erfindungsgemäßer Zusammensetzungen sind ethoxilierte Produkte der Formel (II), bei denen R³ 8 bis 18 Kohlenstoffatome enthält. X kann in Formel (II) für Wasserstoff, einen Methylrest, Ethylrest oder Phenylrest stehen. Es müssen mindestens 50 bis 100 % aller in Komponente B anwesenden Reste X für Wasserstoff stehen, damit die geforderte Primärbenetzbarkeit erzielt wird. Dies bedeutet, daß der Rest X für Wasserstoff steht, wenn Komponente B nur eine einzige Art ethoxilierter Moleküle der Formel (II) enthält. Nachdem jedoch aus technischen und ökonomischen Gründen Komponente B praktisch immer ein Gemisch von Verbindungen ist, die der Formel (II) entsprechen, können in dem Gemisch Verbindungen vorliegen, bei denen X für Wasserstoff steht und Verbindungen, bei denen X für -CH₃, -C₂H₅ oder -C₆H₅ steht. Mindestens 50 % aller anwesenden Reste X müssen jedoch für Wasserstoff stehen. Eine bevorzugte Ausführungsform erfindungsgemäßer Zusammensetzungen besteht jedoch darin, daß alle in Komponente B anwesenden Reste X für Wasserstoff stehen, auch dann, wenn B ein Gemisch ist.

Als Komponente B geeignete Produkte der Formel (II) sind im Handel erhältlich. Sie lassen sich in bekannter Weise herstellen durch Umsetzung der entsprechenden Alkohole R³-OH mit Ethylenoxid und ggf. nachfolgender teilweiser Veretherung der endständigen OH-Gruppe.

Komponente C ist, ohne ein ausgeprägtes Antischaummittel zu sein, eine schaumdämpfende Komponente und als Bestandteil erfindungsgemäßer Zusammensetzungen nötig. Zusammensetzungen, welche eine Komponente A und eine Komponente B, aber keine Komponente C enthalten, können zu Problemen insbesondere dann führen, wenn die Vorbehandlung als Diskontinue-Prozeß in einer Jet-Apparatur durchgeführt werden soll. Es kann dann nämlich zu einem unakzeptabel hohen Schaumniveau kommen.
Komponente C ist ein Alkoxilat der Formel (III) oder ein Gemisch solcher Alkoxilate

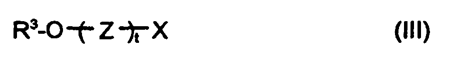

Hierbei besitzen R³ und X die oben im Zusammenhang mit Komponente B genannten Bedeutungen. Ebenso wie Komponente B ist auch Komponente C im Normalfall ein Gemisch von Produkten. Bei der Ethoxilierung/Propoxilierung von Alkoholen R³-OH entstehen wie im Fall der reinen Ethoxilierung Produktgemische. Die einzelnen Verbindungen dieser Gemische der Flormel (III) unterscheiden sich im Wert von t und/oder der Anzahl der Polyoxyethylen- und Polyoxypropyleneinheiten. Femer können - abhängig von den Herstellungsbedingungen - die Verbindungen der Formel (III) als Block-Copolymere oder als random-Copolymere vorliegen. Blockpolymere entstehen z.B. dann, wenn man R³-OH zuerst nur mit Ethylenoxid umsetzt und dann - nach Verbrauch des Ethylenoxids - nur mit Propylenoxid. Diese Art von Blockpolymeren ist als Komponente C erfindungsgemäßer Zusammensetzungen bevorzugt, ggf. nach einer teilweisen Veretherung der endständigen OH-Gruppe. Geeignet sind aber auch Random-Polymere, die durch Umsetzung von R³-OH mit einem Gemisch aus Ethylenoxid und Propylenoxid entstehen bzw. teilweise endständig veretherte Random-Polymere. Wie im Fall von Komponente B müssen 50 bis 100 %, vorzugsweise 100 %, aller in Komponente C vorliegenden Reste X für Wasserstoff stehen, 0 bis 50 % der Reste X können für -CH₃, -C₂H₅ oder -C₆H₅ stehen.
Der Ethoxilierungs-/Propoxilierungsgrad von Komponente C beträgt 4 bis 30, d.h. in Formel (III) steht t für eine Zahl von 4 bis 30. Vorzugsweise ist t eine Zahl von 6 bis 18. Die Gruppe Z in Formel (III) steht für einen zweiwertigen Polyoxyalkylenrest. 20 bis 80 % aller in Komponente C anwesenden Reste Z stehen für den von Ethylenoxid abgeleiteten Rest -CH₂CH₂-O-, die übrigen Reste Z, also 80 - 20 % stehen für den von Propylenoxid abgeleiteten Rest -CHR⁴-CHR⁵-O-. Hierbei steht einer der Reste R⁴ und R⁵ für Wasserstoff, der andere für -CH₃.
In dem Gemisch von Verbindungen, aus dem Komponente C normalerweise besteht. können zwar einzelne Moleküle vorliegen, in denen sich nur -CH₂CH₂-O- Einheiten zwischen R³O und X befinden bzw. einzelne Moleküle, die nur Polyoxypropylen- aber keine Polyoxyethyleneinheiten enthalten. Dies ist bei der technischen Ethoxilierung/Propoxilierung unvermeidbar. Der Anteil solcher Moleküle ist jedoch in Komponente C sehr gering, während der weitaus überwiegende Anteil, d.h. mehr als 90 % von Molekülen in Komponente C sowohl Polyoxyethylen- als auch Polyoxypropyleneinheiten enthält.

Wie im Fall von Komponente B, so lassen sich auch als Komponente C geeignete Produkte auf dem Markt erhalten oder nach allgemein bekannten Methoden herstellen.

Erfindungsgemäße Zusammensetzungen enthalten ferner Wasser (Komponente D).

Die Herstellung erfindungsgemäßer Zusammensetzungen kann im Normalfall problemlos durch Mischen der Komponenten A bis D sowie ggf. weiterer gewünschter Komponenten in beliebiger Reihenfolge unter Rühren bei Raumtemperatur geschehen. In Einzelfällen kann die Einhaltung einer bestimmten Mischungsreihenfolge und/oder eine Temperaturerhöhung Vorteile bezüglich Lagerstabilität bringen. Diese Aussagen gelten auch, falls erfindungsgemäße Zusammensetzungen weitere Komponenten enthalten sollen, z.B. die unten näher beschriebenen Komponenten E und/oder F.

In einer bevorzugten Ausführungsform enthalten erfindungsgemäße Zusammensetzungen außer den Komponenten A bis D, die immer anwesend sein müssen, noch eine Komponente E und/oder eine Komponente F. Sie können an Stelle einer einzigen Verbindung auch Gemische von Verbindungen enthalten, welche unter die unten angegebene Definition von Komponente E und/oder F fallen.
Komponente E ist ein Magnesium- oder ein Calciumsalz, vorzugsweise ein anorganisches, wasserlösliches Salz. Besonders geeignet ist MgCl₂. Die Anwesenheit eines solchen Salzes kann die Eignung erfindungsgemäßer Zusammensetzungen für übliche Vorbehandlungsprozesse erhöhen, bei denen die Textilien mit H₂O₂ gebleicht werden. Diese Prozesse erfordern Stabilisatoren für Wasserstoffperoxid, wozu die genannten Mg- oder Ca-Salze dienen können.

Komponente F ist ein Alkalimetallsalz oder Ammoniumsalz eines Schwefelsäuremonoesters der Formel (V)

R⁸-O-SO₃H (V)

also ein Salz der Formel

R⁸-O-SO₃^{Θ}M^{⊕}, worin M für Na, K oder NH₄ steht. In dieser Formel steht R⁸ für einen linearen oder verzweigten Alkylrest mit 4 bis 12, vorzugsweise 6 bis 10, Kohlenstoffatomen. Der Alkohol R⁸-OH, von dem sich dieses Monoestersalz ableitet, kann ein primärer, sekundärer oder tertiärer Alkohol sein, d.h. die Gruppe -O-SO₃^{Θ}M^{⊕} kann an einem beliebigen Kohlenstoffatom des linearen oder verzweigten einwertigen Rests R⁸ vorliegen.
Die Anwesenheit einer Komponente F in erfindungsgemäßen Zusammensetzungen kann dazu dienen, die Stabilität dieser wäßrigen Zusammensetzungen zu erhöhen.

In einer bevorzugten Ausführungsform enthalten erfindungsgemäße Zusammensetzungen die genannten Komponenten in solchen Mengen, daß pro 100 Gewichtsteile Wasser (Komponente D) folgende Mengen an den Komponenten A, B, C, E und F vorliegen:
5 bis 35 Gewichtsteile an Komponente A, vorzugsweise 10 bis 25 Gew.teile
10 bis 40 Gewichtsteile an Komponente B, vorzugsweise 15 bis 35 Gew.teile
3 bis 30 Gewichtsteile an Komponente C, vorzugsweise 5 bis 25 Gew.teile
0 bis 30 Gewichtsteile an Komponente E, vorzugsweise 2 bis 20 Gew.teile
0 bis 20 Gewichtsteile an Komponente F, vorzugsweise 2 bis 10 Gew.teile

Falls gewünscht, können erfindungsgemäße Zusammensetzungen weitere Zusätze enthalten, insbesondere im Hinblick auf spezielle Vorbehandlungsverfahren und -anforderungen. Solche Zusätze können beispielsweise sein Enzyme, Komplexiermittel oder weitere Tenside; sie können in den für die Vorbehandlungsverfahren üblichen Mengen verwendet werden. Es empfiehlt sich jedoch, vor Verwendung solcher Zusätze im Einzelfall zu prüfen, ob nicht dadurch die oben genannten Vorteile erfindungsgemäßer Zusammensetzungen in einem unakzeptablen Ausmaß verringert werden.

Erfindungsgemäße Zusammensetzungen eignen sich ausgezeichnet zur Behandlung von Fasermaterialien, insbesondere zur Vorbehandlung textiler Fasermaterialien in Form von Geweben oder Gewirken. Die Fasermaterialien können hierbei textile Flächengebilde sein, die aus Cellulose, regenerierter Cellulose oder synthetischen Polymeren bestehen oder die Mischungen solcher Fasern sind. Besonders geeignet sind erfindungsgemäße Zusammensetzungen für die Vorbehandlung textiler Flächengebilde, die zu 70 bis 100 Gewichtsprozent aus Baumwolle bestehen. Der Rest der Fasern besteht hierbei beispielsweise aus Synthetics. Eine Vorbehandlung von textilen Flächengebilden aus Rohbaumwolle mit erfindungsgemäßen Zusammensetzungen führt zu ausgezeichneten Ergebnissen bezüglich Primär- und Wiederbenetzbarkeit bei einer Schaumbildungstendenz, die ein akzeptables Ausmaß auch ohne Verwendung hocheffektiver Antischaummittel wie Silikone nicht überschreitet. Aus diesen Gründen sind erfindungsgemäße Zusammensetzungen auch für Diskontinue-Vorbehandlungsverfahren, z.B. in Jet-Apparaturen, hervorragend geeignet.

Die Applikation erfindungsgemäßer Zusammensetzungen auf das Textilmaterial kann nach bei Vorbehandlungsprozessen üblichen Methoden erfolgen, z.B. durch Tauchapplikation, Foulardieren usw. Zweckmäßigerweise weisen die wäßrigen Flotten, welche zur Vorbehandlung dienen, Konzentrationen auf, wie sie üblich sind, z.B. von 0,03 bis 1 Gewichtsprozent an der Summe der Komponenten A, B und C, bezogen auf Gesamtflotte.
Nach der Vorbehandlung wird das Textilmaterial in üblicher, bekannter Weise, weiterbehandelt, z.B. gefärbt, ggf. nach vorhergehender Trocknung.

Die Erfindung wird nunmehr durch Ausführungsbeispiele veranschaulicht.

Es wurden Zusammensetzungen gemäß nachfolgender Tabelle I hergestellt, wobei die Zahlenangaben unter den jeweiligen Beispielen jeweils den Anteil des betreffenden Bestandteils in Gew% bedeuten.

**Tabelle I**

| Bestandteil | Beispiel 1 | Beispiel 2 | Beispiel 3 | Beispiel 4 | Beispiel 5 | Beispiel 6 | Beispiel 7 |
|---|---|---|---|---|---|---|---|
| a) | 6 | - | 6 | 6 | 6 | 6 | 6 |
| b) | 12 | 12 | 12 | 17 | - | - | 12 |
| c) | 6 | 6 | 6 | 6 | 18 | 6 | 6 |
| d) | 25 | 25 | - | 30 | 25 | 25 | - |
| e) | 10 | 10 | 35 | - | 10 | 10 | 10 |
| f) | - | - | - | - | - | 12 | - |
| g) | - | - | - | - | - | - | 25 |
| Wasser | 41 | 47 | 41 | 41 | 41 | 41 | 41 |
| a) = Magnesiumchloridhexahydrat (Komponente E) | | | | | | | |
| b) = Natriumcumolsulfonat (Komponente A) | | | | | | | |
| c) = Methyl-pentan 1.5-diol (Komponente A) | | | | | | | |
| d) = Gemisch aus ethoxilierten C₁₃-Alkoholen, durchschnittlich 4,6 EO-Einheiten (Komponente B) | | | | | | | |
| e) = ethoxilierter/propoxilierter Alkohol (etwa C₁₂ bis C₁₈), 8 EO, 4 PO, Blockcopolymer (Komponente C) | | | | | | | |
| f) = R'-O SO₃ Na R' = Ethylhexyl (Komponente F) | | | | | | | |
| g) = ethoxilierter C₁₃-Alkohol, durchschnittlich 10 EO-Einheiten | | | | | | | |

Die Beispiele 1, 2, 5 und 6 enthalten sowohl eine Komponente A), als auch eine Komponente B) und eine Komponente C), sind also erfindungsgemäße Beispiele. Die Beispiele 3, 4 und 7 sind nicht-erfindungsgemäße Vergleichsbeispiele, in Beispiel 3 fehlt eine Komponente B, in Beispiel 4 fehlt eine Komponente C, in Beispiel 7 ist der Ethoxilierungsgrad von Bestandteil g) höher als für Komponente B gefordert.

Für jede der Zusammensetzungen Nr. 1 bis 7 wurden Primärbenetzbarkeit und Wiederbenetzbarkeit sowie Tendenz zur Schaumbildung bestimmt.
Zur Ermittlung der Primärbenetzbarkeit wurden in Anlehnung an die Methode ISO 8022 Gewebeproben aus 100 % Rohbaumwolle geprüft. Bestimmt wurde die Zeit (in Sekunden), in welcher das Gewebe durch die betreffende Zusammensetzung vollständig benetzt war. In der unten aufgeführten Tabelle II bedeuten demnach niedrigere Werte für die Primärbenetzbarkeit eine bessere/schnellere Benetzung.
Zur Ermittlung der Schaumbildungstendenz unter Vorbehandlungsbedingungen wurden in eine Labor-Jet-Apparatur jeweils verdünnte NaOH, H₂O₂, ein Stabilisator für H₂O₂ und ca. 1 g/l einer der Zusammensetzungen 1 bis 7 gegeben. Ferner gab man je eine Probe eines Gewirkes aus 100 % Baumwolle in die Apparatur. Unter mechanischer Bewegung wurde der Inhalt der Apparatur zuerst auf 40°C, dann auf 98°C, geheizt und auf 80°C gekühlt. Bei jeder dieser Temperaturen wurde der Inhalt der Apparatur kurze Zeit belassen, dann wurde die Schaumentwicklung beurteilt.
Zur Bestimmung der Wiederbenetzbarkeit wurden die Gewirkproben behandelt wie oben bei der Bestimmung der Schaumbildung angegeben. Anschließend wurden sie aus der Apparatur genommen und getrocknet. Für die Bestimmung der Wiederbenetzbarkeit wurde die Zeit (in Sekunden) gemessen, in der ein aus einer Tropfpipette stammender Wassertropfen das Gewirke vollständig benetzte.

Die Ergebnisse der Prüfungen zeigt Tabelle II.

**Tabelle II**

| | Beispiel 1 | Beispiel 2 | Beispiel 3 | Beispiel 4 | Beispiel 5 | Beispiel 6 | Beispiel 7 |
|---|---|---|---|---|---|---|---|
| Primärbenetzbarkeit (sec.) | 15 | 14 | 30 | 15 | 15 | 15 | 15 |
| Wiederbenetzbarkeit (sec.) | 1 | 1 | 20 | 3 | 1 | 3 | 20 |
| Schaumbildung | wenig | wenig | mittel | sehr stark | wenig | mittel | sehr stark |

## Patentansprüche

1. Zusammensetzung, welche mindestens die Komponenten A, B, C und D enthält,
wobei Komponente A entweder ein Sulfonat der Formel (I) 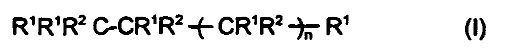 ist,
worin n eine Zahl von 0 bis 8 bedeutet, alle Reste R¹ unabhängig voneinander für Wasserstoff, einen Alkylrest mit 1 bis 4 Kohlenstoffatomen oder einen unsubstituierten oder durch einen Rest der Formel SO₃^{Θ}M^{⊕} substituierten Phenylrest stehen und alle Reste R² unabhängig voneinander für R¹ oder für einen Rest der Formel -SO₃^{Θ}M^{⊕} stehen, wobei Komponente A jedoch mindestens einen Rest der Formel SO₃^{Θ}M^{⊕} enthält und M für Na, K, oder NH₄ steht,
oder wobei Komponente A ein mehrwertiger aliphatischer Alkohol mit 2 bis 12 Kohlenstoffatomen, vorzugsweise mit 4 bis 10 Kohlenstoffatomen, ist,
wobei Komponente B ein ethoxilierter Alkohol der Formel (II) oder ein Gemisch solcher Alkohole 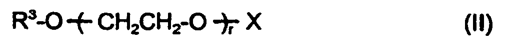 ist,
worin r eine Zahl von 1 bis 8, vorzugsweise von 2 bis 7 ist,
wobei Komponente C ein Alkoxilat der Formel (III) oder ein Gemisch solcher Alkoxilate 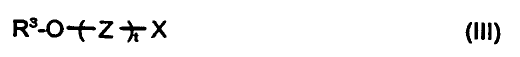 ist,
worin t eine Zahl von 4 bis 30, vorzugsweise von 6 bis 18, ist, 20 bis 80 % aller anwesenden Gruppen Z für -CH₂CH₂-O- und 80 bis 20 % aller anwesenden Gruppen Z
für -CHR⁴-CHR⁵-O- stehen, wobei jeweils einer der Reste R⁴ und R⁵ für Wasserstoff und der andere für CH₃ steht, wobei sowohl in Komponente B als auch in Komponente C R³ für einen linearen oder verzweigten Alkylrest mit 4 bis 20, vorzugsweise 8 bis 18, Kohlenstoffatomen steht und 50 bis 100 %, vorzugsweise 100 %, aller anwesenden X für Wasserstoff und 0 bis 50 %, vorzugsweise 0 %, aller anwesenden Reste X für einen Methyl-, Ethyl- oder Phenylrest stehen,
und wobei Komponente D Wasser ist.

2. Zusammensetzung nach Anspruch 1, **dadurch gekennzeichnet, daß** Komponente A ein Sulfonat der Formel (I) ist, worin mindestens einer aller anwesenden Reste R² für -SO₃^{⊖}M^{⊕} steht.

3. Zusammensetzung nach Anspruch 1 oder 2, **dadurch gekennzeichnet, daß** Komponente A ein Sulfonat der Formel (IV) ist 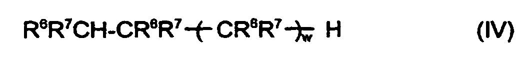 worin w für eine Zahl von 1 bis 3 steht, einer der Reste R⁶ für einen unsubstituierten Phenylrest steht und alle übrigen Reste R⁶ für Wasserstoff stehen, einer der Reste R⁷ für -SO₃^{Θ}M^{⊕} steht und alle übrigen Reste R⁷ für Wasserstoff stehen.

4. Zusammensetzung nach einem oder mehreren der Ansprüche 1 bis 3, **dadurch gekennzeichnet, daß** Komponente A Natriumcumolsulfonat oder Kaliumcumolsulfonat ist.

5. Zusammensetzung nach Anspruch 1, **dadurch gekennzeichnet, daß** Komponente A ein zweiwertiger oder dreiwertiger Alkohol mit 4 bis 8 Kohlenstoffatomen ist.

6. Zusammensetzung nach einem oder mehreren der Ansprüche 1 bis 5, **dadurch gekennzeichnet, daß** sie zusätzlich eine Komponente E und/oder eine Komponente F enthält, wobei Komponente E ein Magnesiumsalz oder ein Calciumsalz, vorzugsweise ein anorganisches, wasserlösliches Salz ist und wobei Komponente F ein Alkalimetallsalz oder Ammoniumsalz eines Schwefelsäuremonoesters der Formel (V)
R⁸-O-SO₃H (V)
ist, wobei R⁸ für einen linearen oder verzweigten Alkylrest mit 4 bis 12, vorzugsweise 6 bis 10, Kohlenstoffatomen steht.

7. Zusammensetzung nach einem oder mehreren der Ansprüche 1 bis 6, **dadurch gekennzeichnet, daß** sie pro 100 Gewichtsteile Wasser (Komponente D) folgende Mengen an den Komponenten A, B, C, E, F enthält:
5 bis 35 Gewichtsteile an Komponente A, vorzugsweise 10 bis 25 Gew.teile
10 bis 40 Gewichtsteile an Komponente B, vorzugsweise 15 bis 35 Gew.teile
3 bis 30 Gewichtsteile an Komponente C, vorzugsweise 5 bis 25 Gew.teile
0 bis 30 Gewichtsteile an Komponente E, vorzugsweise 2 bis 20 Gew.teile
0 bis 20 Gewichtsteile an Komponente F, vorzugsweise 2 bis 10 Gew.teile

8. Verwendung einer Zusammensetzung nach einem oder mehreren der Ansprüche 1 bis 7 zur Behandlung von Fasermaterialien, insbesondere zur Vorbehandlung von textilen Fasermaterialien in Form von Geweben oder Gewirken.

9. Verwendung nach Anspruch 8, **dadurch gekennzeichnet, daß** die Fasermaterialien zu 70 bis 100 Gew% aus Baumwolle bestehen.

## Claims

1. A composition which includes at least the components A, B, C and D,
where component A is either a sulfonate of the formula (I) 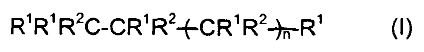 where n is from 0 to 8, any R¹ is independently of the others hydrogen, an alkyl radical of 1 to 4 carbon atoms, an unsubstituted phenyl radical or a phenyl radical substituted by a radical of the formula -SO₃^{⊖}M^{⊕}, and any R² is independently of the others R¹ or a radical of the formula -SO₃^{⊖}M^{⊕}, subject to the proviso that component A contains at least one radical of the formula -SO₃^{⊖}M^{⊕} and M is Na, K or NH₄,
or where component A is a polyhydric aliphatic alcohol of 2 to 12 carbon atoms, preferably of 4 to 10 carbon atoms,
component B is an ethoxylated alcohol of the formula (II) or a mixture of such alcohols 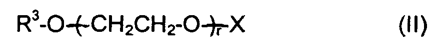 where r is from 1 to 8, preferably from 2 to 7,
component C is an alkoxylate of the formula (III) or a mixture of such alkoxylates 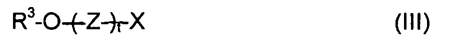 where t is from 4 to 30, preferably from 6 to 18, 20 to 80% of all the Z groups present are -CH₂CH₂-O- and 80 to 20% of all the Z groups present are -CHR⁴-CHR⁵-O-, where in each case one of R⁴ and R⁵ is hydrogen and the other is CH₃, R³ in both component B and component C is a linear or branched alkyl radical of 4 to 20, preferably 8 to 18, carbon atoms and 50 to 100%, preferably 100%, of all the X's present are hydrogen and 0 to 50%, preferably 0%, of all the X's present are a methyl, ethyl or phenyl radical,
and component D is water.

2. A composition according to claim 1, wherein component A is a sulfonate of the formula (I) where at least one of all the R² radicals present is -SO₃^{⊖}M^{⊕}.

3. A composition according to claim 1 or 2, wherein component A is a sulfonate of the formula (IV) 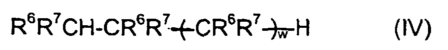 where w is from 1 to 3, one of the R⁶ radicals is an unsubstituted phenyl radical and all the other R⁶ radicals are hydrogen, and one of the R⁷ radicals is -SO₃^{⊖}M^{⊕} and all the other R⁷ radicals are hydrogen.

4. A composition according to one or more of claims 1 to 3, wherein component A is sodium cumenesulfonate or potassium cumenesulfonate.

5. A composition according to claim 1, wherein component A is a dihydric or trihydric alcohol of 4 to 8 carbon atoms.

6. A composition according to one or more of claims 1 to 5, additionally comprising a component E and/or a component F, component E being a magnesium salt or a calcium salt, preferably a water-soluble inorganic salt and component F being an alkali metal salt or ammonium salt of a sulfuric monoester of the formula (V)
R⁸-O-SO₃H (V)
where R⁸ is a linear or branched alkyl radical of 4 to 12, preferably 6 to 10, carbon atoms.

7. A composition according to one or more of claims 1 to 6, including per 100 parts by weight of water (component D) the following amounts of components A, B, C, E, F:
5 to 35 parts by weight of component A, preferably 10 to 25 parts by weight
10 to 40 parts by weight of component B, preferably 15 to 35 parts by weight
3 to 30 parts by weight of component C, preferably 5 to 25 parts by weight
0 to 30 parts by weight of component E, preferably 2 to 20 parts by weight
0 to 20 parts by weight of component F, preferably 2 to 10 parts by weight

8. The use of a composition according to one or more of claims 1 to 7 for treating fiber materials, especially for pretreating textile fiber materials in the form of wovens or knits.

9. The use according to claim 8, wherefor the fiber materials are 70 to 100% by weight cotton.

## Revendications

1. Composition, qui comprend au moins les composants A, B, C et D, dans laquelle le composant A est un sulfonate de formule (I) 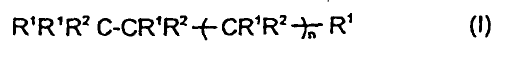 dans laquelle n est un nombre de 0 à 8, tous les groupes R¹ représentent, indépendamment les uns des autres, un atome d'hydrogène, un groupe alkyle contenant 1 à 4 atomes de carbone ou un groupe phényle non substitué ou substitué par un groupe de formule SO₃⁻M⁺, et tous les groupes R² représentent, indépendamment les uns des autres, R¹ ou un groupe de formule SO₃⁻M⁺, le composant A contenant toutefois au moins un groupe de formule SO₃⁻M⁺ et M représentant Na, K ou NH₄,
ou bien le composant A est un polyol aliphatique contenant 2 à 12 atomes de carbone, de préférence 4 à 10 atomes de carbone,
dans laquelle le composant B est un alcool éthoxylé de formule (II) ou un mélange de tels alcools 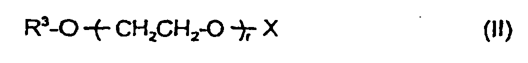 dans laquelle
r est un nombre de 1 à 8, de préférence de 2 à 7,
dans laquelle le composant C est un alcoxylate de formule (III) ou un mélange de tels alcoxylates 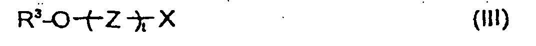 dans laquelle t est un nombre de 4 à 30, de préférence de 6 à 18, 20% à 80% de tous les groupes Z présents représentent un groupe -CH₂CH₂-O- et 80% à 20% de tous les groupes Z présents représentent un groupe -CHR⁴-CHR⁵-O-, dans lequel un des groupes R⁴ et R⁵ représente un atome d'hydrogène et l'autre CH₃,
R³ représente, aussi bien dans le composant B que dans le composant C, un groupe alkyle linéaire ou ramifié contenant 4 à 20, de préférence 8 à 18, atomes de carbone et 50% à 100%, de préférence 100%, de tous les groupes X présents représentent un atome d'hydrogène et 0% à 50%, de préférence 0%, de tous les groupes X présents représentent un groupe méthyle, éthyle ou phényle,
et dans laquelle le composant D est de l'eau.

2. Composition selon la revendication 1, **caractérisée en ce que** le composant A est un sulfonate de formule (I), dans laquelle au moins un parmi tous les groupes R² présents représente un groupe -SO₃⁻M⁺.

3. Composition selon la revendication 1 ou 2, **caractérisée en ce que** le composant A est un sulfonate de formule (IV) 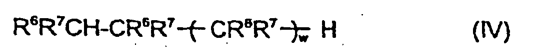 dans laquelle w est un nombre de 1 à 3, un des groupes R⁶ représente un groupe phényle non substitué et tous les autres groupes R⁶ restants représentent un atome d'hydrogène, un des groupes R⁷ représente un groupe -SO₃⁻M⁺ et tous les autres groupes R⁷ restants représentent un atome d'hydrogène.

4. Composition selon l'une ou plusieurs des revendications 1 à 3, **caractérisée en ce que** le composant A est le cumènesulfonate de sodium ou le cumènesulfonate de potassium.

5. Composition selon la revendication 1, **caractérisée en ce que** le composant A est un diol ou un triol contenant 4 à 8 atomes de carbone.

6. Composition selon l'une ou plusieurs des revendications 1 à 5, **caractérisée en ce qu'**elle contient en plus un composant E et/ou un composant F, dans laquelle le composant E est un sel de magnésium ou un sel de calcium, de préférence un sel hydrosoluble inorganique, et dans laquelle le composant F est un sel de métal alcalin ou un sel d'ammonium d'un monoester d'acide sulfurique de formule V
R⁸-O-SO₃H (V)
dans laquelle R⁸ représente un groupe alkyle linéaire ou ramifié contenant 4 à 12, de préférence 6 à 10 atomes de carbone.

7. Composition selon l'une ou plusieurs des revendications 1 à 6, **caractérisée en ce qu'**elle contient, pour 100 parties en poids d'eau (composant D), les quantités suivantes de composants A, B, C, E, F :
5 à 35 parties en poids de composant A, de préférence 10 à 25 parties en poids,
10 à 40 parties en poids de composant B, de préférence 15 à 35 parties en poids,
3 à 30 parties en poids de composant C, de préférence 5 à 25 parties en poids,
0 à 30 parties en poids de composant E, de préférence 2 à 20 parties en poids,
0 à 20 parties en poids de composant F, de préférence 2 à 10 parties en poids.

8. Utilisation d'une composition selon l'une ou plusieurs des revendications 1 à 7 pour le traitement de matières fibreuses, en particulier pour le prétraitement de matières fibreuses textiles sous forme de tissus ou de tricots.

9. Utilisation selon la revendication 8, **caractérisée en ce que** les matières fibreuses sont composées de 70% à 100% en poids de coton.
